# EUROPÄISCHE PATENTSCHRIFT

(11) **EP 2 183 026 B1**
(45) Veröffentlichungstag und Bekanntmachung des Hinweises auf die Patenterteilung: **08.06.2011**
(21) Anmeldenummer: 08802968.1
(22) Anmeldetag: 07.08.2008
(51) Int. Cl.: A61N 5/10, G21F 7/005

(54) **PARTIKELTHERAPIEANLAGE**
PARTICLE THERAPY INSTALLATION
ÉQUIPEMENT DE THÉRAPIE PARTICULAIRE

(30) Priorität: 06.09.2007 DE 102007042336
(43) Veröffentlichungstag der Anmeldung: 12.05.2010
(73) Patentinhaber: Siemens Aktiengesellschaft, 80333 München (DE)
(72) Erfinder: GÜNEYSEL, Murat, 91056 Erlangen (DE)
(86) Internationale Anmeldenummer: PCT/EP2008/060369
(87) Internationale Veröffentlichungsnummer: WO 2009/033897

(56) Entgegenhaltungen:
- WO-A-2004/013865
- WO-A-2005/120640
- WO-A-2007/009786
- US-A- 4 833 335
- US-A1- 2005 029 472
- US-A1- 2007 012 888
- AGOSTEO S ET AL: "Shielding calculations for a 250 MeV hospital-based proton accelerator" NUCLEAR INSTRUMENTS & METHODS IN PHYSICS RESEARCH, SECTION - A:ACCELERATORS, SPECTROMETERS, DETECTORS AND ASSOCIATED EQUIPMENT, ELSEVIER, AMSTERDAM, NL, Bd. A374, Nr. 2, 21. Mai 1996 (1996-05-21), Seiten 254-268, XP004007541 ISSN: 0168-9002
- AGOSTEO S: "Radiation Protection at Medical Accelerators" RADIATION PROTECTION DOSIMETRY, NUCLEAR TECHNOLOGY PUBLISHING, GB, Bd. 96, Nr. 4, 2. Oktober 2001 (2001-10-02), Seiten 393-406, XP009016939 ISSN: 0144-8420

## Beschreibung

Die Erfindung betrifft eine Partikeltherapieanlage, wie sie insbesondere bei der Behandlung von Tumorerkrankungen mit Partikelstrahlen zum Einsatz kommt.

Bei einer Partikeltherapie wird in einem geeigneten Beschleuniger ein Partikelstrahl beispielsweise aus Protonen oder Schwerionen, wie z.B. Kohlenstoffionen, erzeugt. Der Partikelstrahl wird in einem Strahlungskanal geführt und tritt über ein Austrittsfenster des Strahlungskanals in einen Bestrahlungsraum ein, wo die Bestrahlung des Patienten erfolgt. Je nach Ausgestaltung des Bestrahlungsraumes kann dies über ein ortsfestes Strahlaustrittsfenster erfolgen (so genannte "fixed beam"-Bestrahlungsräume), oder aber auch über eine drehbare Gantry, um eine Bestrahlung von mehreren Winkeln aus zu ermöglichen.

Der Kostenaufwand zur Erzeugung und Lenkung eines Partikelstrahls ist im Vergleich zu herkömmlichen Bestrahlungsmethoden wie beispielsweise mit hoch energetischen Röntgenstrahlen deutlich größer. Um dennoch effizient arbeiten zu können, weist eine Partikeltherapieanlage üblicherweise mehrere, in räumliche Nähe zueinander angeordnete Behandlungsräumen auf, in denen jeweils Patienten bestrahlt werden können. Während ein Bestrahlungsvorgang in einem der Behandlungsräume durchgeführt wird, können in den anderen Behandlungsräumen Patienten auf eine folgende Bestrahlungssitzung vorbereitet werden oder im Anschluss an eine erfolgte Bestrahlung versorgt werden. Hierdurch können Zeiten, in denen in einem Bestrahlungsraum keine Bestrahlung erfolgt, sinnvoll genutzt werden.

Die Behandlungsräume sind in einer Partikeltherapieanlage üblicherweise derart angeordnet, dass von einem gemeinsamen Korridor und/oder Vorraum aus der Zutritt zu den Behandlungsräumen ermöglicht wird. Üblicherweise können von diesem Korridor und/oder Vorraum aus weitere Räume betreten werden, die in einer Partikeltherapieanlage zum Einsatz kommen, wie beispielsweise Therapieplanungsräume, Aufenthaltsräume für Patienten bzw. Ärzte, Vorbereitungsräume und/oder Untersuchungsräume für Patienten und ähnliche Räume.

Da in einem Bestrahlungsraum aufgrund der Bestrahlung eine erhebliche Strahlung beispielsweise in Form von hoch energetischer Photonenstrahlung oder Neutronenstrahlung auftreten kann, müssen die Behandlungsräume strahlungsabschirmend aufgebaut sein, derart, dass die übrigen Räume der Partikeltherapieanlage wie beispielsweise der Korridor und/oder Vorraum nicht durch die im Bestrahlungsraum auftretende Strahlung belastet werden.

Dies wird teilweise durch dicke Wände ermöglicht, die einen Bestrahlungsraum zumindest teilweise umgeben. Weiterhin sind Lösungen bekannt, bei denen der Zugang zum Bestrahlungsraum durch einen mäander-förmigen Zugangsweg realisiert ist, in dem teilweise Deckenabhängungen eingezogen sein können. Hierdurch wird die im Bestrahlungsraum entstehende Strahlung gehindert, durch den mäander-förmigen bzw. labyrinth-förmigen Zugangsweg nach draußen zu dringen.

Ein derartiger Zugang zu einem Bestrahlungsraum ist beispielsweise aus der WO 2004/013865 A1 bekannt.

Es ist die Aufgabe der Erfindung, eine Partikeltherapieanlage anzugeben, bei der eine Abschirmung von Behandlungsräumen auf einfache, sichere und kostengünstige Weise gewährleistet werden kann, die eine Platz sparende Anordnung der Behandlungsräume erlaubt und gleichzeitig einen einfachen Zugangsweg zu einem Bestrahlungsraum ermöglicht.

Die Aufgabe wird erfindungsgemäß gelöst durch eine Partikeltherapieanlage nach Anspruch 1. Vorteilhafte Weiterbildungen finden sich in den Merkmalen der abhängigen Ansprüche.

Die Partikeltherapieanlage umfasst:
- zumindest einen Bestrahlungsraum, in dem eine Bestrahlung eines Objektes mit einem Behandlungsstrahl erfolgt,
- einen Vorraum, der vor einer in einem Bestrahlungsraum auftretenden Strahlung abgeschirmt ist und von dem aus ein Zutritt zu dem zumindest einen Bestrahlungsraum erfolgt, und
- zumindest einen Zutrittsbereich zu dem zumindest einen Bestrahlungsraum, der den Bestrahlungsraum mit dem Vorraum verbindet,
wobei in dem Zutrittsbereich eine Strahlenschutztür angeordnet ist.

Der Zutrittsbereich ist dabei derjenige Bereich der einen Zutritt zu dem Bestrahlungsraum ermöglicht. Insbesondere werden im Routinebetrieb Patienten zur Bestrahlung über den Zutrittsbereich in den Raum gefahren werden bzw. nach der Bestrahlung wieder aus dem Raum gefahren. Üblicherweise hat ein Bestrahlungsraum lediglich einen Zutrittsbereich.

Insbesondere ist der Zutrittsbereich in seiner Geometrie derart ausgebildet ist, dass ein Weg, über den eine Patientenliege von dem Vorraum aus zu dem Bestrahlungsraum entlang eines Weges fahrbar ist, im Zutrittsbereich im Wesentlichen geradlinig verläuft.

Es wurde dabei erkannt, dass ein mäander-förmiger bzw. labyrinth-förmiger Zugangsweg, wie er aus dem Stand der Technik bekannt ist, zwar eine wirksame Strahlabschirmung ermöglicht, allerdings auch Nachteile birgt. Zum einen benötigt der mäander-förmige Zugangsweg vergleichsweise viel Platz in einer Partikeltherapieanlage. Da auch ein Zugangsweg von vergleichsweise dicken Strahlenschutzwänden gebildet wird, vergrößert sich die Partikeltherapieanlage und beim Bau fallen insgesamt größere Kosten an. Zum anderen verursacht ein mäander-förmiger Zugangsweg, der üblicherweise mehrere Meter beträgt, eine umständliche Handhabung einer Patientenliege mit einem Patienten, wenn diese von einem Vorraum zu dem Bestrahlungsraum oder umgekehrt gefahren wird.

Diese Probleme werden gelöst, indem im Zutrittsbereich eine Strahlenschutztür angeordnet ist. Die Strahlenschutztür sorgt folglich dafür, dass auch bei einer einfachen Geometrie des Zutrittsbereichs Strahlung nicht von dem Bestrahlungsraum zu anderen Räumen der Partikeltherapieanlage gelangen kann. Hierdurch entfallen mäanderförmige Wegwindungen, deren Zweck es unter anderem ist, die Strahlung abzuschirmen. Die für die Strahlenschutztür anfallenden Kosten werden über den eingesparten Platz durch die einfachere - nun ermöglichte - Bauweise eingespart. Die Strahlenschutztür schirmt diejenige Strahlung ab, die ansonsten bei Zutrittsbereichen mit einfacher Geometrie vermehrt in dem Vorraum gelangen würde.

Die Strahlenschutztür ist dabei insbesondere derart ausgebildet, dass im Behandlungsraum entstehende Strahlung, die durch die Strahlenschutztür durchdringt, um mindestens einen Faktor 50, insbesondere um mindestens einen Faktor 75 und höchst insbesondere um mindestens einen Faktor 100 geschwächt wird. Auf diese Weise ist gewährleistet, dass der Vorraum ausreichend vor Strahlung, z.B. vor bei der Bestrahlung entstehenden schnellen Neutronen und thermischen Neutronen, geschützt wird, selbst wenn im Bestrahlungsraum mit Kohlenstoffionen bestrahlt wird und eine im Vergleich zu Protonen hohe Strahlenbelastung auftritt.

Insbesondere ist die Strahlenschutztür aus Stahl ausgebildet. Stahl eignet sich mit einer Zehntelwertsdicke von 45 cm besonders zur Abschirmung der bei einer Partikeltherapie auftretenden Strahlung. Es können jedoch auch andere Materialien eingesetzt werden. Die Strahlenschutztür kann dabei eine Dicke von mindestens 50 cm, insbesondere von mindestens 75 cm und höchst insbesondere von mindestens 100 cm aufweisen.

Die Geometrie des Zutrittsbereichs ist dabei insbesondere derart ausgebildet, dass der Weg im Zutrittsbereich im Wesentlichen geradlinig verläuft. Unter im Wesentlichen geradlinig wird hierbei ein Weg im Zutrittsbereich verstanden, der so ausgebildet ist, dass eine Patientenliege, wenn sie entlang des Weges im Zutrittsbereich gefahren wird, nur unwesentliche Drehungen von beispielsweise weniger als 40°, insbesondere von weniger als 30°, höchst insbesondere von weniger als 20° und im Idealfall gar keine Drehung ausführen muss. Lediglich bei einem Übertritt von dem Vorraum zu dem Zutrittsbereich bzw. von dem Zutrittsbereich zu dem Bestrahlungsraum sind gegebenenfalls weitere Drehungen durchzuführen.

Dabei ist der Zutrittsbereich bevorzugterweise so ausgebildet, dass nach der Strahlenschutztür eine Drehung der Patientenliege lediglich beim Übertritt von dem Zutrittsbereich zu dem Bestrahlungsraum durchgeführt wird. D.h., dass nachdem eine Patientenliege durch die Strahlenschutztür gefahren wird, soll die Patientenliege - wenn überhaupt - erst dann wieder gedreht werden muss, wenn die Patientenliege von dem Zutrittsbereich in den Bestrahlungsraum, in welchem die eigentliche Bestrahlung stattfindet, gefahren wird. Eine mäanderartige - oder zickzackartige - Fahrweise wird dadurch vermieden, eine kompaktere Bauweise ist möglich.

Der Zutrittsbereich mündet beispielsweise unter einem spitzen Winkel in den Bestrahlungsraum, wodurch sich bereits eine teilweise Strahlungsabschirmung des Zutrittsbereichs von dem Bestrahlungsraum ergibt. Dies ermöglicht einerseits eine Platz sparende Bauweise als auch eine einfache Handhabung einer Patientenliege bei dem Transport durch den Zutrittsbereich.

Der Weg im Zutrittsbereich kann dabei eine Länge von weniger als 10 m und insbesondere von weniger als 8 m haben.

Eine Partikeltherapieanlage weist üblicherweise weitere Behandlungsräume auf, die jeweils über einen eigenen Zutrittsbereich von dem Vorraum aus erreichbar sind. Jeder dieser Zutrittsbereiche kann gemäß den beschriebenen Ausgestaltungen mit einer Strahlenschutztür und einem geraden Zugangsweg ausgebildet werden. Insgesamt werden sowohl eine Strahlabschirmung, eine kostengünstige Herstellung aufgrund einer Platz sparenden Bauweise und eine einfache Handhabung einer Patientenliege bei dem Zugang zu jeden der so ausgebildeten Behandlungsräume und Zutrittsbereiche gewährleistet.

In einer vorteilhaften Ausführungsform ist der Zutrittsbereich zum Bestrahlungsraum derart räumlich angeordnet, dass eine Patientenliege bei einem Übergang von dem Zutrittsbereich zu dem Bestrahlungsraum eine Drehung durchführen muss. Auf diese Weise wird erreicht, dass weniger Strahlung von dem Bestrahlungsbereich im Bestrahlungsraum in den Zutrittsbereich gelangt, wodurch die in Zutrittsbereich angeordnete Strahlenschutztür einfacher und kostengünstiger ausgebildet werden kann.

In einer vorteilhaften Ausführungsform ist zwischen dem Bestrahlungsraum und dem Zutrittsbereich eine strahlungsabschirmende Wand angeordnet, durch die die Strahlenschutztür vor einer direkten Bestrahlung geschützt ist, die von einem Bestrahlungszentrum im Bestrahlungsraum ausgeht. Auch auf diese Weise kann die Strahlenschutztür schlanker dimensioniert werden, da ein Teil der Strahlung durch die strahlungsabschirmende Wand abgeschirmt wird.

In einer weiteren Ausführungsform weist der Bestrahlungsraum einen Wandvorsprung auf, derart, dass sich eine Nische bildet, in der bei der Bestrahlung erzeugte und ausgesendete Neutronen gefangen werden.

Die Strahlenschutztür kann dabei insbesondere als Schiebetür ausgebildet sein, wodurch sich eine einfache Handhabung der Schiebetür und ein schnelles Öffnen bzw. Schließen der Schiebetür realisieren lässt.

Der Vorraum, der üblicherweise als ein Korridor ausgebildet ist, ist dabei üblicherweise so ausgebildet, dass von ihm ein direkter Zutritt zu weiteren Räumen und oder Gängen der Partikeltherapieanlage möglich ist. Beispielsweise können von dem Vorraum aus weitere Behandlungsräume jeweils über einen Zutrittsbereich erreichbar sein. Aber auch andere Räume der Partikeltherapieanlage wie beispielsweise Therapieplanungsräume, Aufenthaltsräume für Patienten bzw. Ärzte, Vorbereitungsräume und/oder Untersuchungsräume für Patienten oder weitere Gänge der Partikeltherapieanlage können von dem Vorraum aus in direkter Weise erreichbar sein.

Der Vorraum muss dabei nicht zwangläufig als ein einzelner, durch Wände abgeschlossener Raum ausgebildet sein. Beispielsweise kann der Vorraum mit anderen Gängen/Räumen einer Partikeltherapieanlage ohne eine trennende Tür in Verbindung stehen. In anderen Anordnungen der Partikeltherapieanlage kann der Vorraum selbst in mehrere kleinere Räume bzw. Gänge unterteilt sein.

Zusätzlich zur Strahlenschutztür kann der Zutrittsbereich von dem Vorraum durch eine zusätzliche Türe getrennt ist, die dünner als die Strahlenschutztür ausgebildet ist. Die zusätzliche Tür kann beispielsweise eine Polyethylen-Tür (PE-Tür) sein, die ggf. Bor umfassen kann. Hierdurch lassen sich effektiv evtl. noch vorhandene thermische Neutronen abschirmen. Die schnellen Neutronen lassen sich effektiv durch die Strahlenschutztür abschirmen.

Ausführungsformen der Erfindung mit vorteilhaften Weiterbildungen gemäß den Merkmalen der abhängigen Ansprüche werden in der folgenden Zeichnung näher erläutert, ohne jedoch darauf beschränkt zu sein. Es zeigen:
- Figur 1: einen schematischen Überblick über eine aus dem Stand der Technik bekannte Partikeltherapieanlage,
- Figur 2: einen schematischen Überblick über einen Bestrah- lungsraum mit einem Zutrittsbereich, der einen gera- den Zugangsweg aufweist.

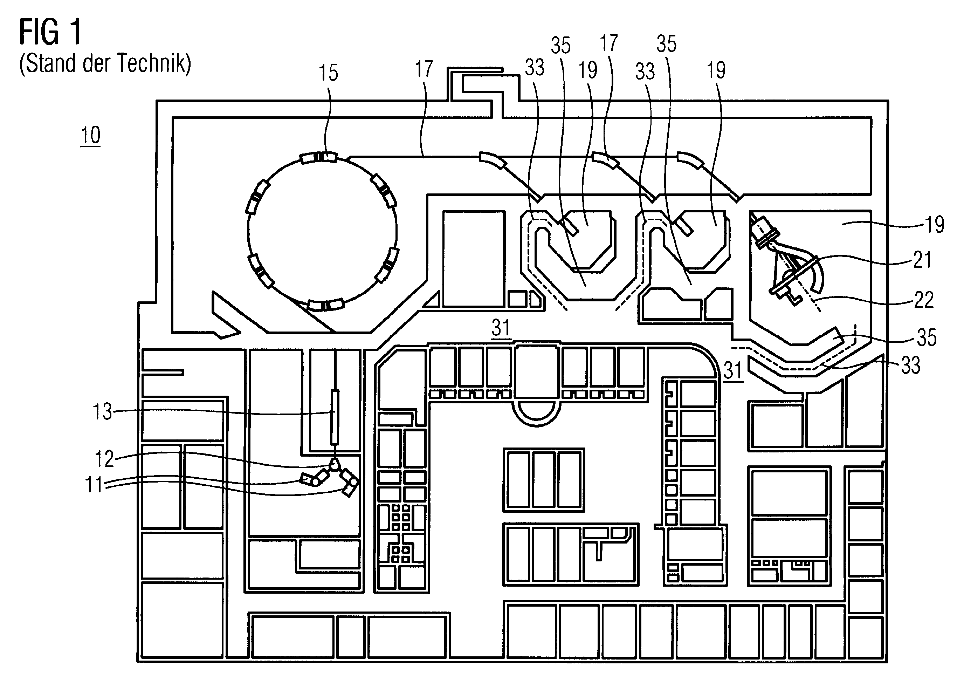

Figur 1 zeigt einen schematischen Überblick über den Aufbau einer Partikeltherapieanlage 10, wie er aus dem Stand der Technik bekannt ist. In einer Partikeltherapieanlage 10 erfolgt eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

In Figur 1 dargestellt sind insgesamt drei Behandlungsräume 19. Zu den Behandlungsräumen 19 gelangt man von einem gemeinsamen, als Korridor ausgebildeten Vorraum 31. Der Zutritt zu den Behandlungsräumen 19 erfolgt dabei jeweils über einen Zutrittsbereich 33. Eine Patientenliege, die von dem Vorraum 31 zu einem der Behandlungsräume 19 gefahren wird, muss entlang eines gewundenen bzw. mäander-förmigen Weges - gestrichelt dargestellt - im Zutrittsbereich 33 gefahren werden. Erst nachdem eine Patientenliege entlang der meterlangen Wege gefahren worden ist, erreicht eine Patientenliege den Bestrahlungsraum 19. Die Behandlungsräume 19 sind dabei von teilweise meterdicken Strahlenschutzwänden umgeben, so dass keine Strahlung aus den Behandlungsräumen 19 in den Vorraum 31 dringt. Durch die mäander-förmigen Zutrittsbereiche 33 ist ebenfalls gewährleistet, dass keine Strahlung über die Zutrittsbereiche 33 nach draußen dringt. Die Zutrittsbereiche 33 benötigen jedoch viel Platz, der von Strahlenschutzwänden umgeben werden muss, so dass diese Lösung vergleichsweise hohe Kosten verursacht.

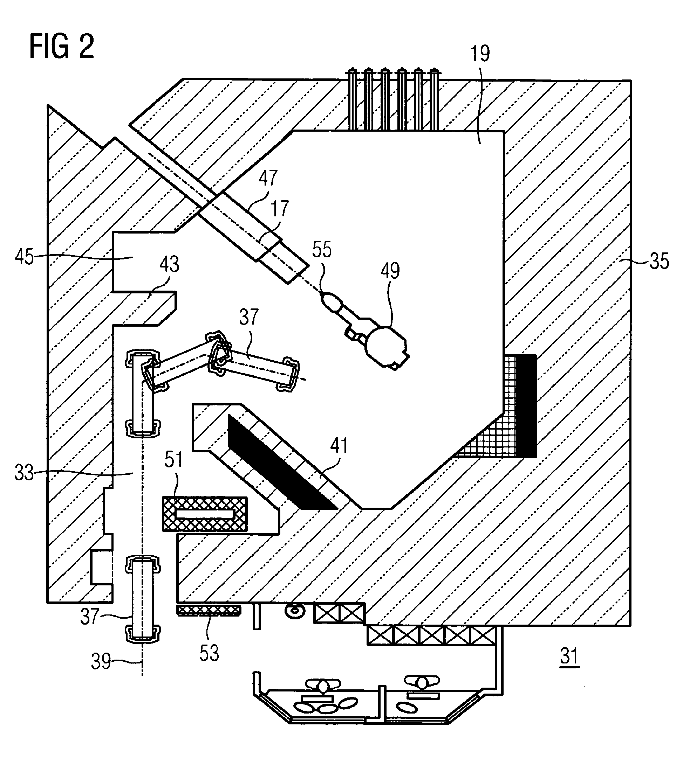

Figur 2 zeigt eine Ausgestaltung eines Bestrahlungsraumes 19 gemäß einer Ausführungsform der Erfindung. In Figur 3 ist lediglich der Bestrahlungsraum 19 mit umgebenden Strukturen näher dargestellt. Eine Partikeltherapieanlage mit einem derartigen Bestrahlungsraum 19 kann ansonsten analog zu der in Figur 1 gezeigten Partikeltherapieanlage aufgebaut sein.

Der Bestrahlungsraum 19 ist dabei derjenige Bereich, der von Strahlenschutzwänden 35 umgeben ist, und in dem eine Positionierung eines zu behandelnden Patienten und dessen Bestrahlung erfolgt. Die Positionierung kann dabei beispielsweise mit einer als Roboterarm 49 ausgebildeten Positioniervorrichtung erfolgen. Ein auf einer Patientenliege 37 liegende Patient wird dabei über den Roboterarm 49 entsprechend einem Behandlungsplan gegenüber einem Strahlaustrittkanal 47 in einer gewünschten Position positioniert. Ein Behandlungsstrahl - hier strichpunktiert angedeutet - gelangt von dem Hochenergie-Strahltransportsystem 17 zu dem Strahlaustrittkanal 47, tritt dort aus und trifft auf das zu bestrahlende Objekt.

Auch bei dem in Figur 3 gezeigten Bestrahlungsraum 19 erfolgt ein Zutritt von einem Vorraum aus über einen Zutrittsbereich 33.

Der Weg 39, entlang dessen eine Patientenliege im Zutrittsbereich 33 gefahren wird, ist dabei im Wesentlichen gerade ausgebildet. In Figur 3 ist dieser Weg 39 gestrichelt eingezeichnet. Eine Drehung der Patientenliege erfolgt lediglich im Übergangsbereich von dem Zutrittsbereich 33 zu dem Bestrahlungsraum 19. Damit keine Strahlung von dem Bestrahlungsraum 19 über den Zutrittsbereich 33 trotz der einfachen Geometrie nach draußen in den Vorraum 31 dringt, ist im Zutrittsbereich 33 eine Strahlenschutztür 51 angeordnet. Sie kann in den Zutrittsbereich 33 derart gefahren werden, dass der Bestrahlungsraum 19 und zu großen Teilen auch der Zutrittsbereich 33 von den anderen Räumen der Partikeltherapieanlage - wie beispielsweise der Vorraum 31 - strahlenschutztechnisch abgeschirmt werden kann. Während eines Bestrahlungsvorganges ist somit sichergestellt, dass der Vorraum 31 und andere Räume der Partikeltherapieanlage 10 vor der im Bestrahlungsraum 19 entstehenden Strahlung geschützt sind. Die hier gezeigte Strahlenschutztür 51 weist eine Dicke von 1 m auf, bei einer Breite von 2 m.

Durch die Strahlenschutztür 51 ist es möglich, den Zutrittsbereich 33 mit einer einfachen Geometrie auszugestalten, so dass ein im Wesentlichen gerade verlaufende Weg 39 im Zutrittsbereich möglich ist, der im Vergleich zu mäander-förmigen bzw. labyrinth-förmigen Zutrittsbereiche auch wesentlich kürzer ausgebildet ist.

Zwischen dem Vorraum 31 und dem Eingang in den Zutrittsbereich 33 kann eine weitere Tür 53 angeordnet sein. Dies hat den Vorteil, dass der Zutrittsbereich auch dann verschlossen werden kann, ohne dass die vergleichsweise große und schwere Strahlenschutztür 51 bewegt werden muss. Zudem kann die weitere Tür noch thermische Neutronen abschirmen.

Zwischen dem Bestrahlungsraum 19 und den Zutrittsbereich 33 ist eine weitere strahlungsabschirmende Wand 41 angeordnet, so dass eine im Bestrahlungszentrum 55 entstehende Strahlung nicht direkt auf die Strahlenschutztür 51 treffen kann. Die strahlungsabschirmende Wand 41 bildet gleichzeitig eine Nische, in die die Strahlenschutztür 51 positioniert werden kann, wenn der Zutrittsbereich 33 geöffnet sein soll. Die Strahlenschutztür 51 ist dabei als Schiebetür ausgebildet. Die strahlungsabschirmende Wand 41 bildet weiterhin einen Teil der Begrenzung des Ganges des Zutrittsbereichs 33.

Weiterhin weist der Bestrahlungsraum 19 einen Mauervorsprung 43 auf, durch den eine Nische 45 gebildet wird, die dazu dient, Neutronen, die während der Bestrahlung entstehen und die in einem Winkel von 60° zur Strahlachse gestreut werden, zu fangen und zu verhindern, dass diese Neutronen auf weitere Gegenstände treffen bzw. nach draußen gelangen.

## Patentansprüche

1. Partikeltherapieanlage, aufweisend:
- zumindest einen Bestrahlungsraum (19), in dem eine Bestrah-lung eines Objektes mit einem Behandlungsstrahl erfolgt,
- einen Vorraum (31), der vor einer in dem Bestrahlungsraum (19) auftretenden Strahlung abgeschirmt ist, und
- einen Zutrittsbereich (33) zu dem Bestrahlungsraum, der den Bestrahlungsraum (19) mit dem Vorraum (31) verbindet, so-dass ein Zutritt von dem Vorraum (31) zu dem Bestrahlungsraum (19) über den Zutrittsbereich (33) erfolgt, wobei in dem Zutrittsbereich eine strahlenschütztür (51) angeordnet ist wobei zwischen dem Bestrahlungsraum (19) und dem Zutrittsbereich (33) eine strahlungsabschirmende Wand (41) angeordnet ist, durch die die Strahlenschutztür (51) vor einer direkten Bestrahlung geschützt ist, die von einem Bestrahlungszentrum (55) im Bestrahlungsraum (19) ausgeht,
**dadurch gekennzeichnet,**
**dass** der Zutrittsbereich (33) in seiner Geometrie derart ausgebildet ist, dass ein Weg (39), entlang dessen eine Patien-tenliege (37) von dem Vorraum (31) aus zu dem Bestrahlungsraum (19) fahrbar ist, dergestalt ist, dass er im Wesentlichen geradlinig verläuft, so dass eine Patientenliege, wenn sie entlang des Weges im Zutrittsbereich gefahren wird, Drehungen von weniger als 40° ausführen muss und weitere Drehungen lediglich bei einem Übertritt von dem Vorraum zu dem Zutrittsbereich bzw. von dem Zutrittsbereich zu dem Bestrahlungsraum gegebenenfalls durchzuführen sind.

2. Partikeltherapieanlage nach Anspruch 1,
**dadurch gekennzeichnet,**
**dass** in dem Bestrahlungsraum (19) ein Wandvorsprung (43) angeordnet ist, derart, dass sich eine Nische (45) bildet, in der bei der Bestrahlung erzeugte und ausgesendete Neutronen gefangen werden.

3. Partikeltherapieanlage nach einem der Ansprüche 1 bis 2,
**dadurch gekennzeichnet,**
**dass** die Strahlenschutztür (51) die im Bestrahlungsraum entstehende und die Strahlenschutztür durchdringende Strahlung um mindestens den Faktor 50, insbesondere um mindestens den Faktor 75 und höchst insbesondere um den Faktor 100 schwächt.

4. Partikeltherapieanlage nach einem der Ansprüche 1 bis 3,
**dadurch gekennzeichnet,**
**dass** die Strahlenschutztür (51) eine Dicke von mindestens 50 cm, insbesondere von mindestens 75 cm und höchst insbesondere von mindestens 100 cm aufweist.

5. Partikeltherapieanlage nach einem der Ansprüche 1 bis 4,
**dadurch gekennzeichnet,**
**dass** die Strahlenschutztür (51) als Schiebetür ausgebildet ist.

6. Partikeltherapieanlage nach einem der Ansprüche 1 bis 5,
**dadurch gekennzeichnet,**
**dass** der Zutrittsbereich (33) von dem Vorraum (31) durch eine zusätzliche Türe (53) getrennt ist, die dünner als die Strahlenschutztür (51) ausgebildet ist.

7. Partikeltherapieanlage nach einem der Ansprüche 1 bis 6,
**dadurch gekennzeichnet,**
**dass** der Vorraum (31) derart ausgebildet ist, dass von dem Vorraum (31) aus ein direkter Zutritt zu weiteren Räumen und/oder Gängen der Partikeltherapieanlage (10) möglich ist.

## Claims

1. A particle therapy installation, featuring:
- at least one irradiation chamber (19), in which an object is irradiated with a treatment beam,
- an antechamber (31) which is shielded from radiation occurring in the irradiation chamber (19), and
- an access area (33) to the irradiation chamber which connects the radiation chamber (19) to the antechamber (31) so that access from the antechamber (31) to the irradiation chamber (19) is via the access area (33),
with a radiation protection door (51) being arranged in the access area,
with a radiation-protection wall (41) being arranged between the irradiation chamber (19) and the access area (33) by which the radiation protection door (51) is protected from direct radiation which goes out from the irradiation center (55) in the irradiation chamber (19),
**characterised in that**
the access area (33) is embodied in its geometry such that a path (39) along which a patient bed (37) can move from the antechamber (31) to the irradiation chamber (19) is such that it is essentially rectilinear, so that a patient bed, when it is moved along the path in the access area, must make turns of less than 40° and further turns are only to be made where necessary when moving from the antechamber to the access area or from the access area to the irradiation chamber respectively.

2. Particle therapy installation according to claim 1,
**characterised in that**
a wall projection (43) is arranged in the irradiation chamber (19) such that it forms a niche (45) in which the neutrons generated and emitted during irradiation are captured.

3. Particle therapy installation according to one of claims 1 to 2,
**characterised in that**
the radiation protection door (51) attenuates the radiation arising in the radiation chamber and penetrating the radiation protection door by at least a factor of 50, especially by at least a factor of 75 and most especially by a factor of 100.

4. Particle therapy installation according to one of claims 1 to 3,
**characterised in that**
the radiation protection door (51) has a thickness of at least 50 cm, especially of at least 75 cm and most especially of at least 100 cm.

5. Particle therapy installation according to one of claims 1 to 4,
**characterised in that**
the radiation protection door (51) is embodied as a sliding door.

6. Particle therapy installation according to one of claims 1 to 5,
**characterised in that**
the access area (33) is separated from the antechamber (31) by an additional door (53) which is embodied thinner than the radiation protection door (51).

7. Particle therapy installation according to one of claims 1 to 6,
**characterised in that**
the antechamber (31) is embodied such that direct access is possible from the antechamber (31) to further rooms and/or corridors of the particle therapy installation (10).

## Revendications

1. Installation de thérapie particulaire, comprenant :
- au moins une chambre d'irradiation (19) dans laquelle se déroule une irradiation d'un objet au moyen d'un faisceau d'irradiation,
- une antichambre (31) qui est protégée d'un rayonnement produit dans la chambre d'irradiation (19) par un blindage et
- une zone d'accès (33) menant à la chambre d'irradiation, qui relie la chambre d'irradiation (19) à l'antichambre (31), de manière à ce que l'on accède de l'antichambre (31) à la chambre d'irradiation (19) par la zone d'accès (33), une porte de protection contre les rayons (51) étant située dans la zone d'accès, une cloison anti-rayonnement (41) étant située entre la chambre d'irradiation (19) et la zone d'accès (33), cloison qui protège la porte de protection contre les rayons (51) d'un rayonnement direct provenant d'un centre d'irradiation (55) dans la chambre d' irradiation (19),
**caractérisée en ce que** la zone d'accès (33) a une configuration géométrique telle qu'un chemin (39) le long duquel un lit de patient (37) peut être amené de l'antichambre (31) à la chambre d'irradiation (19) s'étend de manière essentiellement rectiligne, de manière à ce qu'un lit de patient n'ait besoin d'effectuer que des rotations de moins de 40° lorsqu'il emprunte le chemin dans la zone d'accès et à ce que d'autres rotations ne soient nécessaires que lors d'un passage de l'antichambre à la zone d'accès resp. de la zone d'accès à la chambre d'irradiation, le cas échéant.

2. Installation de thérapie particulaire selon la revendication 1, **caractérisée en ce que** la chambre d'irradiation (19) comporte une saillie de paroi (43) située de manière à former une niche (45) dans laquelle des neutrons produits et émis lors de l'irradiation sont interceptés.

3. Installation de thérapie particulaire selon l'une des revendications 1 à 2, **caractérisée en ce que** la porte de protection contre les rayons (51) atténue le rayonnement qui est produit dans la chambre d'irradiation et qui passe à travers la porte de protection contre les rayons d'un facteur d'au moins 50, notamment d'un facteur d'au moins 75 et, notamment, en particulier du facteur 100.

4. Installation de thérapie particulaire selon l'une des revendications 1 à 3, **caractérisée en ce que** la porte de protection contre les rayons (51) a une épaisseur d'au moins 50 cm, notamment d'au moins 75 cm et, notamment, en particulier d'au moins 100 cm.

5. Installation de thérapie particulaire selon l'une des revendications 1 à 4, **caractérisée en ce que** la porte de protection contre les rayons (51) est exécutée en tant que porte coulissante.

6. Installation de thérapie particulaire selon l'une des revendications 1 à 5, **caractérisée en ce que** la zone d'accès (33) est séparée de l'antichambre (31) par une porte supplémentaire (53) qui est moins épaisse que la porte de protection contre les rayons (51).

7. Installation de thérapie particulaire selon l'une des revendications 1 à 6, **caractérisée en ce que** l'antichambre (31) est exécutée de manière à ce qu'un accès direct soit possible de l'antichambre (31) vers d'autres chambres et/ou couloirs de l'installation de thérapie particulaire (10).
